# EUROPEAN PATENT APPLICATION

(11) **EP 4 670 811 A1**
(43) Date of publication of application: **31.12.2025**
(21) Application number: 24185424.9
(22) Date of filing: 28.06.2024
(51) Int. Cl.: B01D 3/00, B01D 3/40

(54) **SYSTEMS AND METHODS FOR REGENERATING EXTRACTIVE DISTILLATION SOLVENT WITH ENHANCED ENTHALPY**

(71) Applicant: SABIC Global Technologies B.V., 4612 PX Bergen op Zoom (NL)
(72) Inventor: Kommuri, Subbarao, 6160 GA Geleen (NL); Dalal, Anilendu, 6160 GA Geleen (NL)
(74) Representative: Sabic INDIA Intellectual Property Group

(57) **Abstract**

Provided here are systems and methods that include facilitate regeneration of solvents for extractive distillation solvent with enhanced enthalpy. Examples include a method that includes receiving a high-temperature lean solvent stream including 1-methylpyrrolidin-2-one (NMP) and at a temperature between 145 °C and 155 °C from a degasser column. The method includes splitting the high-temperature lean solvent stream into a first portion and a second portion, transferring heat from the first portion to a solvent feed stream to produce a cooled lean solvent stream and a heated solvent feed stream, and regenerating the second portion to produce a purified lean solvent stream. The method includes directing the cooled lean solvent stream and the purified lean solvent stream to an extractive distillation zone.

## Description

### Technical Field

The disclosure relates to the regeneration of solvent used in extractive distillation, including increased enthalpy utilization provided by diverting a high-temperature lean solvent stream to a regeneration unit to facilitate improved solvent regeneration therein.

### Background

Extractive distillation processes employ a solvent to facilitate separation of components in a mixture that may otherwise be inseparable through traditional distillation. For example, butadiene processing plants may use extractive distillation to isolate a desired product stream including buta-1,3-diene from other C₄ hydrocarbon compounds with similar boiling points. In such a process, a solvent stream is circulated through a series of distillation columns to achieve the desired separations, while accumulating various impurities within the solvent stream. These impurities may be removed from the solvent stream via solvent regeneration to avoid processing issues and maintain operating efficiency. However, the feed rate for solvent regeneration may be limited based on an amount of heat transfer available to a regeneration unit.

### Summary

As noted above, existing solvent regeneration systems are limited in the amount of solvent they are able to regenerate based on insufficient heat transfer. As such, a major challenge is identified herein as the improvement of heat transfer for such systems. The present disclosure includes process adjustments that significantly enhance the heat transfer available for solvent regeneration, thereby enabling a direct increase of a feed rate of solvent to a regeneration unit, without additional capital expenditures. For example, the disclosed systems and methods provide enhanced heat transfer in a regeneration unit, which facilitates an increased regeneration processing capacity and correspondingly increased solvent feed rate to the regeneration unit, without requiring additional processing equipment.

In more detail, systems disclosed herein include a split point that receives a high-temperature lean solvent stream produced by a degasser column, a heat exchanger fluidly coupled to receive a first portion of the high-temperature lean solvent stream from the split point, and a regeneration unit fluidly coupled downstream of the split point to receive a second portion of the high-temperature lean solvent stream. As such, the first portion of the lean solvent stream is provided to the heat exchanger to transfer heat to a separate stream directed therethrough, while the second portion of the lean solvent stream retains its enthalpy for utilization within the regeneration unit. Accordingly, present systems and methods improve operations by enabling the heat exchanger and the regeneration unit to be in use simultaneously to process separate portions of the lean solvent stream, thus redirecting enthalpy that is otherwise used in the heat exchanger to facilitate operation of the regeneration unit.

The disclosure herein provides several embodiments and examples of systems for the regeneration of solvent, such as 1-methylpyrrolidin-2-one (NMP), from a butadiene processing system and methods for solvent regeneration. Examples include a method that includes receiving a high-temperature lean solvent stream including NMP and at a temperature between 145 °C and 155 °C from a degasser column. The method includes splitting the high-temperature lean solvent stream into a first portion and a second portion, transferring heat from the first portion to a solvent feed stream to produce a cooled lean solvent stream and a heated solvent feed stream, and regenerating the second portion to produce a purified lean solvent stream. The method includes directing the cooled lean solvent stream and the purified lean solvent stream to an extractive distillation zone.

In some examples, the second portion includes between about 0.1 and 0.5 weight percent (wt. %) of the high-temperature lean solvent stream. In some examples, the temperature is about 150 °C and the high-temperature lean solvent stream includes at least about 90 wt. % NMP. In some examples, the method further includes adjusting a ratio between the first portion and the second portion based on one or more operating parameters of the extractive distillation zone. In some examples, the method further includes detecting an increase in impurities in a monitored solvent stream via one or more sensors and increasing an amount of the second portion relative to the first portion in response to detecting the increase.

In some examples, the method further includes pressurizing the high-temperature lean solvent stream to a threshold pressure of at least about 15 bar absolute. In some examples, the method further includes producing at least one product stream that is rich in buta-1,3-diene within the extractive distillation zone. In some examples, the method further includes degassing a solvent stream to produce the high-temperature lean solvent stream and a vaporous hydrocarbon product stream.

Examples include a system that includes one or more splitting devices operable to receive a high-temperature lean solvent stream from a degasser column and split the high-temperature lean solvent stream into a first portion and a second portion. The high-temperature lean solvent stream includes NMP and is at a temperature between 140 °C and 160 °C. The system includes a heat exchanger operable to transfer heat from the first portion of the high-temperature lean solvent stream to a solvent feed stream and produce a cooled lean solvent stream and a heated solvent feed stream. The system includes a NMP regeneration unit operable to receive the second portion of the high-temperature lean solvent stream and produce a purified lean solvent stream. The cooled lean solvent stream and the purified lean solvent stream are directed to an extractive distillation zone to produce one or more product streams including crude butadiene.

In some examples, the second portion includes between about 0.1 and 0.5 wt. % of the high-temperature lean solvent stream. In some examples, the second portion includes about 0.2 wt. % of the high-temperature lean solvent stream. In some examples, the temperature of the high-temperature lean solvent stream from the degasser column includes about 150 °C and the NMP regeneration unit includes an operating pressure of about 0.1 bar absolute or less.

In some examples, the one or more splitting devices includes a pipe fitting or a manifold. In some examples, the one or more splitting devices includes a control valve, and the system includes a controller communicatively coupled to the one or more control valves. The controller is configured to receive input indicative of an increase in impurities in a monitored solvent stream and increase an amount of the second portion relative to the first portion via adjusting the one or more valves in response to the input. In some examples, the monitored solvent stream includes the high-temperature lean solvent stream or a solvent stream within the extractive distillation zone.

Examples include a system that includes a degasser column operable to receive a solvent stream including NMP and split the solvent stream into at least a vaporous hydrocarbon product stream and a high-temperature lean solvent stream at a temperature between 145 °C and 155 °C. The system includes a heat exchanger operable to transfer heat from a first portion of the high-temperature lean solvent stream to a solvent feed stream and produce a cooled lean solvent stream and a heated solvent feed stream. The system includes a NMP regeneration unit operable to receive a second portion of the high-temperature lean solvent stream and produce a purified lean solvent stream. The cooled lean solvent stream and the purified lean solvent stream are directed to an extractive distillation zone.

In some examples, the second portion includes less than about 0.5 wt. % of the high-temperature lean solvent stream. In some examples, the system includes one or more valves fluidly coupled downstream of the degasser column and operable to adjust a ratio of the first portion to the second portion. In some examples, the system includes the extractive distillation zone, and the extractive distillation zone is operable to produce one or more product streams including crude butadiene based on one or more extractions performed with the cooled lean solvent stream and the purified lean solvent stream. In some examples, the system includes a pump fluidly coupled between the degasser column and the heat exchanger and operable to increase a pressure of the high-temperature lean solvent stream to at least 15 bar absolute.

Still other aspects and advantages of these exemplary embodiments and other embodiments, are discussed in detail herein. Moreover, it is to be understood that both the foregoing information and the following detailed description provide merely illustrative examples of various aspects and embodiments, and are intended to provide an overview or framework for understanding the nature and character of the claimed aspects and embodiments. Furthermore, it is to be understood that the features of the various embodiments described herein are not mutually exclusive and may exist in various combinations and permutations.

### Brief Description of the Drawings

Embodiments will be readily understood by the following detailed description in conjunction with the accompanying drawings. To facilitate this description, like reference numerals designate like structural elements or procedures in a method. Embodiments are illustrated by way of example and not by way of limitation in the accompanying drawings. The present disclosure can be better understood by referring to the following figures. These drawings illustrate the principles of the disclosure and no limitation of the scope of the disclosure is thereby intended.
**FIG. 1** is a schematic representation of a solvent regeneration system including a degasser column that produces a lean solvent stream, a heat exchanger fluidly coupled to receive a first portion of the lean solvent stream, and a regeneration unit fluidly coupled to receive a second portion of the lean solvent stream, according to an example.
**FIG. 2** is a schematic representation of a solvent regeneration system including one or more control valves to control an amount of a lean solvent stream into a first portion directed to a heat exchanger and a second portion directed to a regeneration unit, according to an example.
**FIG. 3** is a schematic representation of a control system for controlling operation of the disclosed systems for solvent regeneration, according to an example.
**FIG. 4** is a block diagram of a method for regenerating a portion of a high-temperature lean solvent stream diverted from upstream of a heat exchanger, according to an example.

### Detailed Description

So that the manner in which the features and advantages of the examples of the systems and methods disclosed herein, as well as others that will become apparent, may be understood in more detail, a more particular description of examples of systems and methods briefly summarized above may be had by reference to the following detailed description of examples thereof, in which one or more are further illustrated in the appended drawings, which form a part of this specification. It is to be noted, however, that the drawings illustrate only various examples of the systems and methods disclosed herein and are therefore not to be considered limiting of the scope of the systems and methods disclosed herein as it may include other effective examples as well.

The description may use the phrases "in some embodiments," "in various embodiments," "in an embodiment," or "in certain embodiments," which may each refer to one or more of the same or different embodiments. Furthermore, the terms "comprising," "including," "having," and the like, as used with respect to embodiments of the present disclosure, are synonymous.

The term "about" refers to a range of values including the specified value, which a person of ordinary skill in the art would consider reasonably similar to the specified value. In embodiments, "about" refers to values within a standard deviation using measurements generally acceptable in the art. In one non-limiting embodiment, when the term "about" is used with a particular value, then "about" refers to a range extending to ±10 % of the specified value, alternatively ± 5 % of the specified value, or alternatively ±1 % of the specified value, or alternatively ± 0.5 % of the specified value. In embodiments, "about" refers to the specified value.

The use of the words "a" or "an" when used in conjunction with any of the terms "comprising," "including," "containing," or "having," in the claims or the specification may mean "one," but it is also consistent with the meaning of "one or more," "at least one," and "one or more than one." The terms "wt. %", "vol. %", or "mol. %" refers to a weight, volume, or molar percentage of a component, respectively, based on the total weight, the total volume of material, or total moles, that includes the component. In a non-limiting example, 10 grams of a component in 100 grams of the material is 10 wt. % of such component. The term "enriched" or "rich" or their variations mean an amount of at least generally about 20 wt. %, and preferably about 25 wt. %, of a compound or class of compounds in a stream. The term "substantially contains" means that the mixture includes at least 60 %, or even at least 70 %, or even at least 80 % by weight of the relevant hydrocarbon-based compounds. The terms "reducing," "reduced," or any variation thereof, when used in the claims and/or the specification includes any measurable decrease or complete removal to achieve a desired result. The terms "increasing," "increased," or any variation thereof, when used in the claims and/or the specification includes any measurable increase to achieve a desired result.

As used herein, the term "zone" can refer to an area including one or more units and/or one or more sub-zones. Units can include one or more reactors or reactor vessels, separators, strippers, extraction columns, fractionation columns, distillation columns, heaters, exchangers, pipes, pumps, valves, compressors, sensors, and controllers. Additionally, a unit, such as a reactor, dryer, or vessel, can further include one or more zones or sub-zones that contain various equipment.

The present disclosure describes various examples related to systems and methods for enhanced solvent regeneration based on increased utilization of the enthalpy of a solvent stream. As described, the solvent stream of certain examples is or includes 1-methylpyrrolidin-2-one (or N-methylpyrrolidone), referred to herein as NMP. The examples included herein provide efficient regeneration of solvent streams that recirculate through extractive distillation zones or sections, such as extractive distillation zones for butadiene processing systems. For example, the disclosed systems and methods provide enhanced heat transfer in a regeneration unit, which facilitates an increased regeneration processing capacity and correspondingly increased solvent feed rate to the regeneration unit, without requiring additional capital expenditure on further processing or heating equipment.

In certain butadiene processing systems, the circulating solvent is regenerated to remove impurities such as process-generated polymers, chemical additives, and/or any other contaminants or residue content buildup within the solvent. The resulting purified solvent thus increases the overall performance and reliability of the butadiene processing. That is, solvent regeneration provides increased operational stability and continuity to the overall system, while preventing fouling issues. As examples, the increased solvent processing efficiency provided herein can reduce or eliminate problems encountered in certain butadiene processing systems, such as bottleneck conditions and/or excessively high polymer buildup that is treated with an increased solvent processing rate.

The systems and methods disclosed herein enable improved regeneration of solvents, such as NMP used within a butadiene processing system, via a specifically interconnected arrangement of processing units or zones that diverts a portion of a high-temperature lean solvent stream for regeneration from upstream of a heat exchanger. In one example, a solvent regeneration system includes (i) one or more splitting devices (e.g., control valves, manual valves, pipe fittings, manifold) forming a split point that receives a high-temperature lean solvent stream produced by a degasser column and having a temperature between about 145 degrees Celsius (°C) and about 155 °C, (ii) a NMP-NMP heat exchanger that receives a first portion of the high-temperature lean solvent stream from the split point to heat a separate solvent feed stream and produce a cooled lean solvent stream and a heated solvent feed stream, and (iii) a NMP regeneration unit that receives a second portion of the high-temperature lean solvent stream from the split point and produces a purified lean solvent stream. The NMP-NMP heat exchanger and the NMP regeneration unit are utilized simultaneously to process separate portions of the lean solvent stream, thus redirecting enthalpy that is otherwise used in the heat exchanger to enhance heat transfer in the regeneration unit.

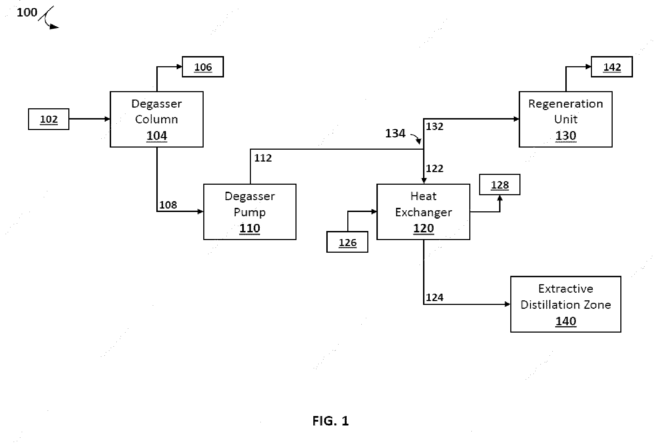

**FIG. 1** is a schematic representation of a system **100** for regenerating solvent used for extractive distillation, such as extractive distillation within butadiene recovery systems. The system **100** of the illustrated example includes a degasser column (or degasser) that produces a high-temperature lean solvent stream, a heat exchanger (or NMP-NMP heat exchanger) fluidly coupled to receive a first portion or fraction of the high-temperature lean solvent stream, and a regeneration unit (or NMP regeneration unit) fluidly coupled to receive a second portion or fraction of the high-temperature lean solvent stream. As such, the first portion of the lean solvent stream is provided to the heat exchanger to transfer heat to a separate stream directed therethrough, while the second portion of the lean solvent stream retains its enthalpy for utilization within the regeneration unit.

In more detail, the system **100** facilitates the regeneration of a solvent stream **102** that is supplied to a degasser column **104.** The solvent stream **102** can be any suitable circulated stream or byproduct stream having a solvent and one or more gasses such as hydrocarbon compounds dissolved in the solvent. The degasser column **104** includes a vessel having an inlet to receive the solvent stream **102** and two or more outlets to output respective portions of the solvent stream **102** that are separated from one another within the degasser column **104.** The degasser column **104** can operate with any suitable operating parameters to split the solvent stream **102** into two or more streams based on differences in physical and/or chemical properties of the chemical compounds of the solvent stream **102.** For example, the degasser column **104** can perform pressure modifications, temperature modifications, ultrasonification, agitation, membrane separation, and/or any other separation processes to remove gas from the solvent stream **102.** In the illustrated example, the degasser column **104** receives the solvent stream **102,** separates the dissolved gasses from the solvent, and outputs a vaporous hydrocarbon product stream **106** from a top outlet and outputs a lean solvent stream **108** (or low-pressure, high-temperature solvent stream) from a bottom outlet. The vaporous hydrocarbon product stream **106** is directed to any desired additional separation units and/or processing units to yield one or more hydrocarbon products, in some examples. In certain examples, the degasser column **104** also includes a third outlet to output an additional solvent stream that is rich in acetylenes, which can be condensed and supplied by the system **100.**

In some examples, the lean solvent stream **108** or solvent thereof is or includes NMP. In certain examples, the solvent is a mixture of NMP and water. The solvent is at least about 90 weight percent (wt. % NMP) and less than about 10 wt. % water, in some examples. For example, the solvent can be about 91.7 wt. % NMP and 8.3 wt. % water. The degasser column **104** of certain examples maintains a temperature at its bottom that is in a range between about 145 degrees Celsius (°C) and about 155 °C. In some examples, the bottom of the degasser column **104** is about 150 °C. In some examples, the lean solvent stream **108** provided from the bottom of the degasser column **104** is at the same temperature in the range between about 145 °C and about 155 °C, such as about 150 °C.

In the system **100,** the degasser column **104** is fluidly coupled to a degasser pump **110** that receives and pressurizes the lean solvent stream **108** to a desired pressure or threshold pressure. That is, the degasser pump **110** includes an inlet fluidly coupled to the bottom outlet of the degasser column **104.** The degasser pump **110** can be any suitable type of pump for pressurizing a fluid, such as a centrifugal pump, a positive displacement pump, and so forth. In an example, the degasser pump **110** increases a pressure of the lean solvent stream **108** to at least about 15 bar absolute (bar(a)) or bar. In another example, the degasser pump **110** increases a pressure of the lean solvent stream **108** to between about 10 and about 20 bar(a). In certain examples, the degasser pump **110** increases a pressure of the lean solvent stream **108** to at least about 5, 10, 15, 20, or 25 bar(a). The degasser pump **110** thus outputs a high-temperature lean solvent stream **112** (or high-pressure, high-temperature lean solvent stream) having an increased pressure relative to the lean solvent stream **108.** In some examples, the degasser pump **110** is or includes multiple pumps that are fluidly coupled downstream of the degasser column **104** in any suitable series and/or parallel arrangement.

In the illustrated example, an outlet of the degasser pump **110** for outputting the high-temperature lean solvent stream **112** is fluidly coupled to both a first inlet of a heat exchanger **120** and an inlet of a regeneration unit **130.** For example, a single conduit is fluidly coupled to the outlet of the degasser pump **110** and fluidly coupled to a first conduit branch and a second conduit branch. The high-temperature lean solvent stream **112** is therefore split into a first lean solvent stream **122** (or first portion of the high-temperature lean solvent stream **112**) supplied along the first conduit branch to the heat exchanger **120** and a second lean solvent stream **132** (or second portion of the high-temperature lean solvent stream **112**) supplied along the second conduit branch to the regeneration unit **130.** The region, area, or zone at which the high-temperature lean solvent stream **112** is separated into the first lean solvent stream **122** and the second lean solvent stream **132** is referred to herein as a split point **134** or diversion point. The split point **134** can utilize any suitable piping, joints, tees, fittings, manifolds, manual valves, and/or control valves to divide or split the high-temperature lean solvent stream **112** into any suitably sized streams, in certain examples.

In an example, the split point **134** is designed to split, divide, or separate the high-temperature lean solvent stream **112** into the first lean solvent stream **122** and the second lean solvent stream **132** according to a specified weight percent ratio. For example, the split point **134** can cause the first lean solvent stream **122** to receive 99.5 to 99.9 wt. % and cause the second lean solvent stream **132** to receive the remaining 0.5 to 0.1 wt. % of the high-temperature lean solvent stream **112.** For example, the second lean solvent stream **132** can include between about 0.1 and 0.5 wt. % of the high-temperature lean solvent stream **112.** In an example, the second lean solvent stream **132** includes about 0.2 wt. % of the high-temperature lean solvent stream **112.** In other words, the split point **134** can provide a split ratio of the first lean solvent stream **122** to the second lean solvent stream **132** of 99.5 : 0.5, 99.6 : 0.4, 99.7 : 0.3, 99.8 : 0.2, 99.9 : 0.1 or any other suitable ratio between the first lean solvent stream **122** and the second lean solvent stream **132.**

In certain examples, the split point **134** includes one or more controllable valves or adjustable flow control elements to enable modification of the division of the high-temperature lean solvent stream **112,** as discussed in more detail with reference to later figures. In general, the amount of the high-temperature lean solvent stream **112** diverted to form the second lean solvent stream **132** can be particularly selected based on one or more monitored operating conditions of the system **100.** For example, operating conditions that affect a selected weight percent of the high-temperature lean solvent stream **112** provided to form the second lean solvent stream **132** can include an amount or concentration of impurities detected in one or more lean or rich solvent streams, an amount of heat transfer occurring in the regeneration unit **130,** an amount of heat transfer occurring in the heat exchanger **120,** and so forth.

Fluidly coupled downstream of the split point **134,** the heat exchanger **120** is operable within the system **100** to transfer heat or enthalpy from the first lean solvent stream **122** to another solvent stream within the system **100.** For example, the heat exchanger **120** includes the first inlet to receive the first lean solvent stream **122** and includes a first outlet to output a cooled lean solvent stream **124,** having a lower temperature than the first lean solvent stream **122.** The cooled lean solvent stream **124** is supplied from the heat exchanger **120** to an extractive distillation zone **140** of the system **100,** in which it is utilized for extractive distillation of butadiene (e.g., buta-1,3-diene) and/or any other suitable hydrocarbon products.

Additionally, the heat exchanger **120** includes a second inlet to receive a solvent feed stream **126** and a second outlet to output a heated solvent feed stream **128,** having a higher temperature than the solvent feed stream **126.** The heated solvent feed stream **128** receives the enthalpy transferred from the first lean solvent stream **122** and is directed to another portion of the system **100** or another portion of the extractive distillation zone **140** to perform additional operations therein. In certain examples, the solvent feed stream **126** is a solvent stream that is rich in hydrocarbons. For example, the heat exchanger **120** can receive the solvent feed stream **126** from a bottom of a rectifier column positioned within the extractive distillation zone **140.** In some examples, the heated solvent feed stream **128** is provided back to the same rectifier column of the extractive distillation zone **140** to utilize the additional enthalpy provided from the first lean solvent stream **122.**

The heat exchanger **120** can be or include any suitable equipment for enabling indirect heat transfer between the two streams directed therethrough. As non-limiting examples, the heat exchanger **120** can be a shell and tube heat exchanger, a plate heat exchanger, or any other heat exchanger type, including any suitable flow arrangement and number of passes. The first inlet and the first outlet of the heat exchanger **120** are fluidly coupled by a first flow path through the heat exchanger **120,** and the second inlet and the second outlet are fluidly coupled by a second flow path through the heat exchanger **120.** In an example, the first flow path is provided within a tube-side of the heat exchanger **120** and the second flow path is provided within a shell-side of the heat exchanger **120.** In another example, the first flow path is provided within the shell-side of the heat exchanger **120** and the second flow path is provided within the tube-side of the heat exchanger **120.**

The regeneration unit **130** is also fluidly coupled downstream of the split point **134,** in a parallel flow arrangement with the heat exchanger **120.** In certain examples, the regeneration unit **130** is operable within the system **100** to regenerate and purify the solvent of the second lean solvent stream **132.** As such, the regeneration unit **130** includes a vessel with the inlet to receive the second lean solvent stream **132** and with an outlet to output a purified lean solvent stream **142.** The purified lean solvent stream **142** output by the regeneration unit **130** is free or substantially free of polymers, chemical additives, and/or any other contaminants or residue content. The purified lean solvent stream **142** is thus directed to another portion of the system **100** or to the extractive distillation zone **140** to perform extractive distillation of butadiene and/or any other suitable hydrocarbon products. The extractive distillation zone **140** thus operates to produce one or more product streams including buta-1,3-diene based on one or more extractions performed with the cooled lean solvent stream **124** and the purified lean solvent stream **142.** For example, the one or more product streams may be rich in buta-1,3-diene. In certain examples, the one or more product streams include crude butadiene (or a mixture of buta-1,3-diene and other hydrocarbons), which is further purified to isolate pure or substantially pure buta-1,3-diene. In some examples, the purified lean solvent stream **142** is supplied to a cooled lean solvent circulation system and/or directed to a main washer or extractive distillation column of the extractive distillation zone **140.** In certain examples, the main washer also outputs the solvent stream **102** that is supplied to the degasser column **104.**

Within the regeneration unit **130,** the solvent can be purified by heating that vaporizes pure or substantially pure solvent such as NMP, while relatively heavier impurities remain within the vessel of the regeneration unit **130.** Accordingly, certain examples include periodically or intermittently shutting down the regeneration unit **130** to clean and remove accumulated impurities therein. In some examples, the regeneration unit **130** processes the solvent with a heat duty of about 90 kilowatts (kW). In certain examples, the heat duty of the regeneration unit **130** is between about 80 and 100, 80 and 90, 85 and 90, 90 and 95, or 90 and 100 kW. However, it should be understood that the present techniques are applicable for increasing an available solvent processing rate of any suitable regeneration unit. In some examples, the regeneration unit **130** includes an operating pressure that is about 0.05 bar(a). In some examples, the regeneration unit **130** includes an operating pressure that is less than about 0.10 bar(a).

Compared to other systems that may divert solvent to a regeneration unit from a point downstream of the heat exchanger **120,** the various examples of the illustrated system **100** retains additional enthalpy in the second lean solvent stream **132** for use within regeneration processes, such as vaporization of purified solvent. In some examples, the processing capacity of the regeneration unit **130** is limited or constrained by an amount of heat that is input to the regeneration unit. The disclosed examples of the system **100** solve such problems by conserving the thermal energy of the second lean solvent stream **132** that bypasses the heat exchanger **120.** This configuration significantly enhances the heat transfer available for solvent regeneration and provides a direct increase in a feed rate of the second lean solvent stream **132** for improved solvent regeneration, without utilizing additional equipment or expenditures. The examples disclosed herein also operate both the heat exchanger **120** and the regeneration unit **130** concurrently or simultaneously, thus performing both solvent-to-solvent heat exchange and solvent regeneration on the respective portions of high-temperature lean solvent stream **112** directed to these components. This concurrent operation provides efficiency benefits over certain other systems that are limited to performing only one of heat exchange or solvent regeneration at a given time.

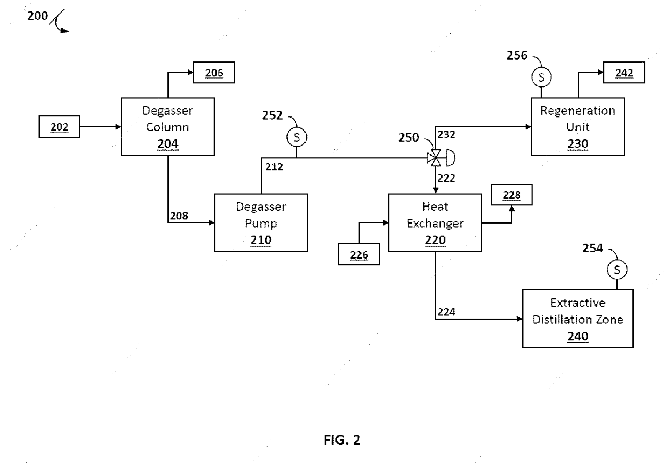

**FIG. 2** is a schematic representation of a system **200** for regenerating solvent including one or more control valves positioned for automatically controlling diversion of solvent to a regeneration unit from upstream of a heat exchanger, according to an example. The system **200** includes a degasser column **204,** a degasser pump **210,** a heat exchanger **220,** a regeneration unit **230,** and an extractive distillation zone **240,** which each correspond to and include similar components as the zones discussed above with respect to **FIG. 1****.** These components are similarly labeled, and their descriptions are not repeated in detail for improved clarity.

As discussed above, a lean solvent stream **208** is produced by the degasser column **204** and pressurized via the degasser pump **210** to form a high-temperature lean solvent stream **212.** A first portion of the high-temperature lean solvent stream **212** is directed to the heat exchanger **220** as the first lean solvent stream **222** and a second portion of the high-temperature lean solvent stream **212** is directed to the regeneration unit **230** as the second lean solvent stream **232.** Additionally, the system **200** includes a control valve **250** positioned and fluidly coupled between the degasser pump **210,** the heat exchanger **220,** and the regeneration unit **230** to actively control the split or division of the high-temperature lean solvent stream **212.** The control valve **250** is illustrated as a three-way control valve, though it should be understood that any other suitable arrangement of valves, tees, fittings, and so forth can be utilized to split the high-temperature lean solvent stream **212** into two portions, fractions, or branches.

The control valve **250** is communicatively coupled to a controller, such as the controller discussed with reference to later figures, which can dynamically actuate the control valve **250** based on continuous monitoring of the system **200.** As an example, the controller can adjust the flow rate of the second lean solvent stream **232** based on sensor signals indicative of an amount or concentration of impurities detected in one or more lean or rich solvent streams, an amount of heat transfer occurring in the regeneration unit **230,** an amount of heat transfer occurring in the heat exchanger **220,** and so forth.

The system **200** further includes multiple sensors to monitor operating conditions and provide sensor signals indicative of the monitored operating conditions to the controller. For example, a first sensor **252** is illustrated as operatively coupled to monitor the high-temperature lean solvent stream **212.** A second sensor **254** is illustrated as operatively coupled to monitor the extractive distillation zone **240** and a third sensor **256** is illustrated as operatively coupled to monitor the regeneration unit **230.** In other examples, any suitable arrangement and distribution of one or more sensors can be implemented to facilitate monitoring of desired operating conditions and operating parameters of the system **200.** The sensors **252, 254, 256** can each be designed to monitor one or more operating conditions, including flow rates, temperatures, pressures, concentrations, and so forth.

Based on sensor signals provided by the sensors **252, 254, 256,** the controller can optimize the split of the high-temperature lean solvent stream **212** for improved solvent regeneration and extractive distillation. As an example, the controller can identify an increase in an amount and/or an increased rate of buildup of impurities in a solvent stream or vessel of the system **200** and correspondingly increase the amount of the high-temperature lean solvent stream **212** that is supplied to the regeneration unit **230.** In another example, the controller can identify a decrease in impurities in a solvent stream or vessel and thus decrease the amount of the high-temperature lean solvent stream **212** that is supplied to the regeneration unit **230.**

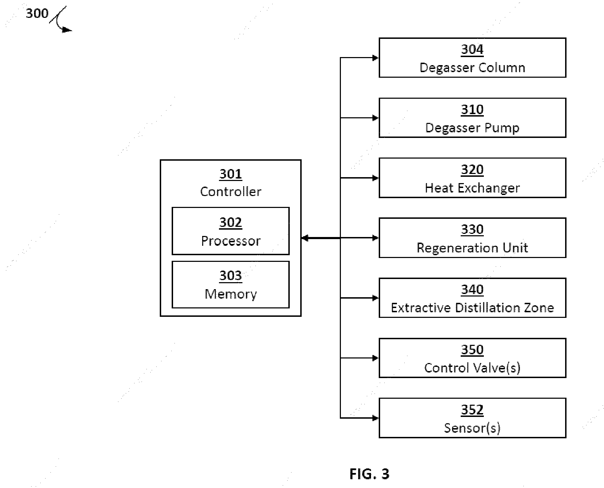

**FIG. 3** is a schematic representation of a control system **300** for controlling the examples of the systems discussed above. The control system **300** includes at least one controller **301.** Each controller **301** includes at least one processor **302,** which may be or include a central processing unit (CPU), a graphics processing unit (GPU), a co-processing unit, a sub-processing unit, or any other suitable electronic data processor. Each controller **301** includes at least one memory **303,** which may be or include random access memory (RAM), read-only memory (ROM), or any other suitable electronic memory or storage. For the illustrated example, the controller **301** is communicatively connected to or in signal communication with each of the components present in a particular implementation of the systems discussed above. For example, the controller **301** can be communicatively coupled to a degasser column **304,** a degasser pump **310,** a heat exchanger **320,** a regeneration unit **330,** an extractive distillation zone **340,** one or more control valves **350,** and/or one or more sensors **352** (e.g., temperature sensors, pressure sensors, flow sensors, content analyzers). The controller **301** can further be communicatively connected to any other elements that are included in or facilitate operation of the systems discussed above.

The communicative connection between the controller **301** and the various components, units, zones, and devices enables the controller **301** to receive monitoring and operational data from sensors **352** and/or sub-controllers of each of these components, units, or zones present in the examples discussed above, and further enables the controller **301** to provide control signals (e.g., electrical signals, instructions, data packets) to modify the operation of each of these components, units, or zones. Moreover, the controller **301** can implement any suitable monitoring, analysis, and/or actuation steps to automatically control extractive distillation, degassing, solvent regeneration, product isolation, and any other processes occurring in a suitable system disclosed herein. For example, the controller **301** may receive monitoring data from the sensors **352** or components of one or more of the degasser column **304,** the degasser pump **310,** the heat exchanger **320,** the regeneration unit **330,** and/or the extractive distillation zone **340,** and based on predefined threshold values for their respective operating parameters, provide suitable control signals to modify their operation or operation of the one or more control valves **350** to ensure that each component, unit, or zone operates in accordance with any predefined threshold values.

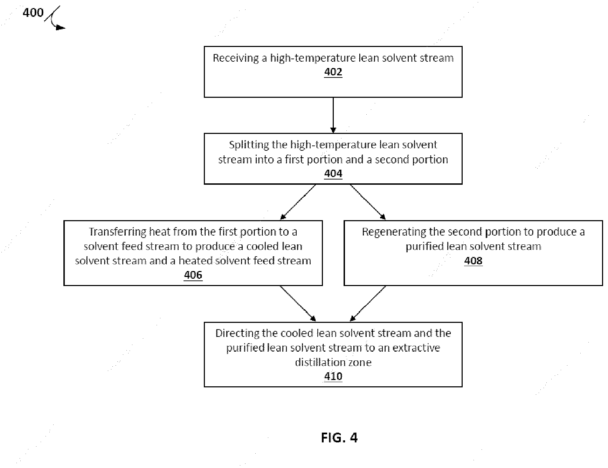

Disclosed examples also include methods for regenerating solvent with enhanced enthalpy utilization within regeneration units. **FIG. 4** is a block diagram a method **400** to regenerate a portion of a high-temperature lean solvent stream diverted from upstream of a heat exchanger, according to an example. In some examples, the method **400** generally corresponds to certain features of the elements and systems discussed with reference to **FIGS. 1-3****.** However, the method **400** may also be performed with other systems that are different in one or more aspects relative to the previously described systems. In some examples, one or more features of the method **400** may be performed or partially performed by a controller (such as a controller of **FIG. 3**) that includes one or more processors that execute machine readable instructions stored on one or more memory devices.

The method includes the step **402** of receiving a high-temperature lean solvent stream. In some examples, the high-temperature lean solvent stream is received from a degasser column, which has a bottom temperature of between 145 °C and 155 °C. The high-temperature lean solvent stream of such examples thus has a temperature between 145 °C and 155 °C. In some examples, a pump or degasser pump can be included downstream of the degasser column to pressurize the high-temperature lean solvent stream to at least a predefined threshold pressure, such as 15 bar(a).

The method includes the step **404** of splitting the high-temperature lean solvent stream into a first portion (or first lean solvent stream) and a second portion (or second lean solvent stream). In some examples, the second portion is a diverted portion or a minor fraction of the high-temperature lean solvent stream, such as between about 0.1 and 0.5 wt. % thereof. The first portion and the second portion can be produced from the high-temperature lean solvent stream via any suitable splitting device or devices discussed above. For example, one or more splitting devices can form a split point that is fluidly coupled downstream of the degasser column (or a pump downstream of the degasser column) and that is fluidly coupled upstream of a heat exchanger and a regeneration unit.

In some examples, the one or more splitting devices include a passive device, such as a pipe fitting, manifold, or manual valve. In some examples, the one or more splitting devices additionally or alternatively include a controllable or actuatable device, such as a control valve communicatively coupled to a controller. In such examples, the controller may further monitor an amount of impurities in one or more monitored solvent streams and adjust the split between the first portion and the second portion based on the amount of impurities. For example, in response to receiving input or sensor signals indicative of an increase in impurities in a monitored solvent stream (e.g., after shutdown and cleaning of the regeneration unit), the controller can increase an amount of the second portion relative to the first portion via adjusting one or more control valves.

Moreover, the method includes the step **406** of transferring heat from the first portion to a solvent feed stream to produce a cooled lean solvent stream and a heated solvent feed stream. The method includes the step **408** of regenerating the second portion to produce a purified lean solvent stream. As illustrated, steps **406** and **408** can be performed concurrently or simultaneously. That is, the first portion and the second portion of the high-temperature lean solvent stream can both flow at the same time to the heat exchanger and the regeneration unit, respectively. This method thus enables the solvent regeneration to be performed continuously, with enhanced enthalpy provided to the regeneration unit. Additionally, the method includes the step **410** of directing the cooled lean solvent stream and the purified lean solvent stream to an extractive distillation zone, in which the streams can perform suitable extractive distillation for product recovery.

### Examples

The following examples are put forth so as to provide those of ordinary skill in the art with a complete disclosure and description of how the compounds, compositions, articles, devices and/or methods claimed herein are made and evaluated and, therefore, are intended to be purely exemplary and are not intended to limit the disclosure. Efforts have been made to ensure accuracy with respect to numbers (e.g., amounts, temperature, etc.), but some deviations should be accounted for. There are numerous variations and combinations of reaction conditions, for example, component concentrations, desired solvents, solvent mixtures, temperatures, pressures and other reaction ranges and conditions that can be used to optimize results obtained from the described process. Only reasonable and routine experimentation will be required to optimize such process conditions.

### Example 1: Increased solvent processing capacity

The systems and methods disclosed herein can regenerate solvent streams at improved processing rates, based on enthalpy conserved in a lean solvent stream supplied to a regeneration unit. Simulations were performed to confirm the benefits of the disclosed systems and methods of solvent regeneration for butadiene processing systems.

As a non-limiting example, a reference system was considered that generally includes a degasser column, a degasser pump, a heat exchanger, and a regeneration unit. A lean solvent stream is output by the degasser column and supplied through the degasser pump and through the heat exchanger to produce a cooled lean solvent stream. Then, a first portion of the cooled lean solvent stream is directed to an extractive distillation zone and a second portion of the cooled lean solvent stream is supplied to the regeneration unit. The second portion of the cooled lean solvent stream includes a lower temperature than the lean solvent stream present upstream of the heat exchanger. As such, the regeneration unit of the reference system receives a reduced amount of thermal energy compared to the presently disclosed systems.

A tested system was also considered that includes the degasser column, the degasser pump, the heat exchanger, and the regeneration unit. Similar to the examples discussed above, the tested simulated system includes a split point upstream instead of downstream of the heat exchanger. This arrangement enables a portion of the lean solvent stream that is directed to the regeneration unit to maintain the thermal energy it contained at the exit of the degasser column, while simultaneously enabling heat transfer in the heat exchanger with a remaining portion of the lean solvent stream.

Solvent regeneration operations were simulated for each of the reference system and the tested system. The operating parameters included a solvent composition of 91.7 wt. % NMP and 8.3 wt. % water, a degasser column bottom temperature of 150 °C, a degasser pump outlet pressure of 15 bar(a), a regeneration unit vessel pressure of 0.05 bar(a), and a regeneration unit heat duty of 89 kW. For the reference system, the lean solvent stream traverses the heat exchanger before entering the regeneration unit at a temperature of 95 °C. In contrast, the lean solvent stream entering the regeneration unit in the tested system bypasses the heat exchanger and has maintained a temperature of 150 °C. Simulated operation of the reference system with these operating conditions provides a solvent regeneration rate of 500 kilograms (kg) per hour (h) of NMP. Additionally, simulated operation of the tested system with these operating conditions provides a solvent regeneration rate of 615 kg/h of NMP, which is a 23 % increase in processing capacity compared to the reference system. As such, the presently disclosed systems deliver an increase in solvent processing capacity, without the use of additional regeneration units, heat exchangers, or other complex installations.

When ranges are disclosed herein, ranges from any lower limit may be combined with any upper limit to recite a range not explicitly recited, as well as ranges from any lower limit may be combined with any other lower limit to recite a range not explicitly recited, in the same way, ranges from any upper limit may be combined with any other upper limit to recite a range not explicitly recited. Additionally, reference to values stated in ranges includes each and every value within that range, even though not explicitly recited. Thus, every point or individual value may serve as its own lower or upper limit combined with any other point or individual value or any other lower or upper limit, to recite a range not explicitly recited.

Other objects, features and advantages of the disclosure will become apparent from the foregoing drawings, detailed description, and examples. These drawings, detailed description, and examples, while indicating specific embodiments of the disclosure, are given by way of illustration only and are not meant to be limiting. In further embodiments, features from specific embodiments may be combined with features from other embodiments. For example, features from one embodiment may be combined with features from any of the other embodiments. In further embodiments, additional features may be added to the specific embodiments described herein. It should be understood that although the disclosure contains certain aspects, embodiments, and optional features, modification, improvement, or variation of such aspects, embodiments, and optional features can be resorted to by those skilled in the art, and that such modification, improvement, or variation is considered to be within the scope of this disclosure.

## Claims

1. A method comprising:
receiving a high-temperature lean solvent stream comprising 1-methylpyrrolidin-2-one (NMP) and being at a temperature between 145 °C and 155 °C from a degasser column;
splitting the high-temperature lean solvent stream into a first portion and a second portion;
transferring heat from the first portion to a solvent feed stream to produce a cooled lean solvent stream and a heated solvent feed stream;
regenerating the second portion to produce a purified lean solvent stream; and
directing the cooled lean solvent stream and the purified lean solvent stream to an extractive distillation zone.

2. The method of claim 1, wherein the second portion comprises between about 0.1 and 0.5 weight percent (wt. %) of the high-temperature lean solvent stream.

3. The method of claim 1, wherein the temperature is about 150 °C and the high-temperature lean solvent stream comprises at least about 90 wt. % NMP.

4. The method to any of the claims 1-3, comprising:
adjusting a ratio between the first portion and the second portion based on one or more operating parameters of the extractive distillation zone.

5. The method to any of the claims 1-4, comprising:
detecting an increase in impurities in a monitored solvent stream via one or more sensors; and
in response to detecting the increase, increasing an amount of the second portion relative to the first portion.

6. The method to any of the claims 1-5, comprising:
pressurizing the high-temperature lean solvent stream to a threshold pressure of at least about 15 bar absolute.

7. The method to any of the claims 1-6, comprising:
producing at least one product stream comprising crude butadiene within the extractive distillation zone.

8. The method of to any of the claims 1-7, comprising:
degassing a solvent stream to produce the high-temperature lean solvent stream and a vaporous hydrocarbon product stream.

9. A system comprising:
one or more splitting devices operable to receive a high-temperature lean solvent stream from a degasser column and split the high-temperature lean solvent stream into a first portion and a second portion, the high-temperature lean solvent stream comprising 1-methylpyrrolidin-2-one (NMP) and being at a temperature between 140 °C and 160 °C;
a heat exchanger operable to transfer heat from the first portion of the high-temperature lean solvent stream to a solvent feed stream and produce a cooled lean solvent stream and a heated solvent feed stream; and
a NMP regeneration unit operable to receive the second portion of the high-temperature lean solvent stream and produce a purified lean solvent stream, wherein the cooled lean solvent stream and the purified lean solvent stream are directed to an extractive distillation zone to produce one or more product streams comprising buta-1,3-diene.

10. The system of claim 9, wherein the second portion comprises between about 0.1 and 0.5 weight percent (wt. %) of the high-temperature lean solvent stream.

11. The system to any of the claims 9-10, wherein the second portion comprises about 0.2 wt. % of the high-temperature lean solvent stream.

12. The system to any of the claims 9-11, wherein the one or more splitting devices comprises a pipe fitting or a manifold.

13. The system to any of the claims 9-12, wherein the one or more splitting devices comprises a control valve, and wherein the system comprises a controller communicatively coupled to the one or more control valves and configured to:
receive input indicative of an increase in impurities in a monitored solvent stream; and
increase an amount of the second portion relative to the first portion via adjusting the one or more valves in response to the input.

14. The system to any of the claims 9-13, wherein the monitored solvent stream comprises the high-temperature lean solvent stream or a solvent stream within the extractive distillation zone.

15. A system comprising:
a degasser column operable to receive a solvent stream comprising 1-methylpyrrolidin-2-one (NMP) and split the solvent stream into at least a vaporous hydrocarbon product stream and a high-temperature lean solvent stream with a temperature between 145 °C and 155 °C;
a heat exchanger operable to transfer heat from a first portion of the high-temperature lean solvent stream to a solvent feed stream and produce a cooled lean solvent stream and a heated solvent feed stream; and
a NMP regeneration unit operable to receive a second portion of the high-temperature lean solvent stream and produce a purified lean solvent stream, wherein the cooled lean solvent stream and the purified lean solvent stream are directed to an extractive distillation zone.
